# EUROPEAN PATENT APPLICATION

(11) **EP 2 762 573 A1**
(43) Date of publication of application: **06.08.2014**
(21) Application number: 13154103.9
(22) Date of filing: 05.02.2013
(51) Int. Cl.: C12Q 1/68

(54) **In vitro method for determining cholestatic properties of a compound.**

(71) Applicant: Rikilt, 6708 WB Wageningen (NL)
(72) Inventor: Peijnenburg, Adrianus Antonius Cornelis Maria, 6701 CL WAGENINGEN (NL); Szalowska, Ewa, 6843 RP ARNHEM (NL)
(74) Representative: Habets, Winand

(57) **Abstract**

The invention is in the field of medical diagnostics and molecular biology. It provides markers that may be used in an in vitro method for determining whether a compound has cholestatic properties. More in particular, the invention relates to an in vitro method for determining whether a compound has cholestatic properties, comprising the step of exposing a liver cell to the compound, incubating the liver cell with the compound for an appropriate amount of time, determining the expression level of genes Bcl2l11, Parp 16, Mras, Myo1e, Nme6 ABCG5, ABCG8, SERPINE1, BAAT, and KLF15 in the liver cell, wherein an aberrant expression of more than 4 of these genes indicates that the compound has cholestatic properties.

## Description

### Field of the invention

The invention is in the field of medical diagnostics and molecular biology. It provides markers that may be used in an in vitro method for determining whether a compound has cholestatic properties.

### Background of the invention

Drug induced liver injury is still a major problem in medical care and pharmacological industry. One example of severe liver damage is cholestasis (Padda et al. 2011) that comprises approximately 17% of all hepatic adverse drug reactions (Velayudham and Farrell 2003). Cholestasis results in a dramatic increase in liver and serum bile acids that eventually can lead to liver failure (Woolbright and Jaeschke 2012). Several currently used drugs have been shown to induce cholestasis in humans and rats, e.g. cyclosporine A (CsA) used as an immunosuppressant (Chan et al. 1998; Dandel et al. 2010; Deters et al. 1997; Rotolo et al. 1986), chlorpromazine (CPZ) applied as an antipsychotic (Abernathy et al. 1992; Akerboom et al. 1991) and ethinyl estradiol (EE) (Lindberg 1992; Paulen et al. 2010; Zhao et al. 2009) used as a compound of contraceptive pills. Bile acids (BA) are amphipathic molecules synthesized from cholesterol in hepatocytes, which together with phospholipids and cholesterol are actively secreted by the liver as bile.

Upon a meal ingestion bile is released into the intestinal lumen to enable absorption of dietary lipids and fat-soluble vitamins (Lefebvre et al. 2009). BA levels are tightly regulated because high BAs concentrations may become cytotoxic (Lefebvre et al. 2009).

There is a lack of in vitro models that accurately predict development of drug induced cholestasis in humans. Cell line models e.g.HepG2 or mouse primary hepatocytes are commonly used to detect drugs with cholestatic properties.

The spectrum of drug-induced cholestasis ranges from 'bland' reversible cholestasis to chronic forms due to the vanishing bile duct syndrome. Agents known for many years to cause cholestasis include estrogens and anabolic steroids, chlorpromazine, erythromycin, and the oxypenicillins; structurally similar congeners of these drugs (tamoxifen, newer macrolides) may also cause cholestasis. Contemporary drugs linked to cholestastic liver injury include ticlopidine, terfenadine, terbinafine, nimesulide, irbesartan, fluoroquinolones, cholesterol-lowering 'statins,' and some herbal remedies (greater celandine, glycyrrhizin, chaparral). Amoxillin-clavulanate, ibuprofen, and pediatric cases of the vanishing bile duct syndrome are recent additions to a long list of drugs associated with the vanishing bile duct syndrome. Particular human leukocyte antigen profiles have recently been identified among those who have developed cholestasis with specific drugs (tiopronin and amoxicillin-clavulanate)

In the development of new drugs, it is imperative that unwanted side-effects are recognized as early as possible. Hence, there is an urgent need for a reliable test method that identifies compounds with cholestatic properties in an easy, fast and economic way.

### Summary of the invention

It was found that a liver cell responded with an altered level of certain genes when it was exposed to a compound with cholestatic properties.

The invention therefore provides an in vitro method for determining whether a compound has cholestatic properties, comprising the step of exposing a liver cell to the compound, incubating the liver cell with the compound for an appropriate amount of time, determining the expression level of genes Bcl2l11, Parp 16, Mras, Myo1e, Nme6 ABCG5, ABCG8, SERPINE1, BAAT, and KLF15 in the liver cell, wherein an aberrant expression of more than 4 of these genes indicates that the compound has cholestatic properties.

### Detailed description of the invention

Liver cells were exposed to a variety of compounds as described in the examples section. As pro-cholestatic model compounds CyclosporinA (CsA) and chlorpromazine (CPZ) were selected. Tetracycline (TETRA), valproic acid (VA), and amiodarone (AMIOD) were used as model steatotic drugs. As a model for necrotic drugs acetaminophen (APAP), paraquat (PQ) and isoniazid (ISONZ) were used at a maximum, non-cytotoxic dose. Both steatotic and necrotic compounds could be regarded as negative controls i.e. non-cholestatic compounds Five mice were used in 5 separate experiments as described in the Examples section.

Quantitative PCR test were developed as described in the Examples section. Relative expression levels (relative to actin B expression levels) were determined for 10 genes as listed in table 1.

**Table 1; Marker genes for cholestasis**

| **Gene** | **Function** |
|---|---|
| BAAT | The protein encoded by this gene catalyzes the transfer of the bile acid moiety from the acyl-CoA thioester to either glycine or taurine, the second step in the formation of bile acid-amino acid conjugates which serve as detergents in the gastrointestinal tract. |
| ABCG8 | The protein encoded by this gene functions as a half-transporter to limit intestinal absorption and promote biliary excretion of sterols. |
| ABCG5 | The protein encoded by this gene functions as a half-transporter to limit intestinal absorption and promote biliary excretion of sterols. |
| KLF15 | KLF15 is increased by fasting and decreased by feeding. Plays a role in regulation of energy metabolism and gluconeogenesis. |
| SERPINE1 | SERPINE1=PAI-1 is a serine protease inhibitor that functions as the principal inhibitor of tissue plasminogen activator (tPA) and urokinase (uPA), the activators of plasminogen, contributes to cholestatic injury in mice. |
| Nme6 | NME6 is ubiquitous enzyme that catalyze transfer of gamma-phosphates, via a phosphohistidine intermediate, between nucleoside and dioxynucleoside tri- and diphosphates |
| Parp16 | Poly (ADP-ribose) polymerase (PARP) catalyzes the post-translational modification of proteins by the addition of multiple ADP-ribose moieties. PARP transfers ADP-ribose from nicotinamide dinucleotide (NAD) to glu/asp residues on the substrate protein, and also polymerizes ADP-ribose to form long/branched chain polymers. PARP inhibitors are being developed for use in a number of pathologies including cancer, diabetes, stroke and cardiovascular disease. |
| Myo1e | This gene encodes a member of the nonmuscle class I myosins which are a subgroup of the unconventional myosin protein family. The unconventional myosin proteins function as actin-based molecular motors. Class I myosins are characterized by a head (motor) domain, a regulatory domain and a either a short or long tail |
| | domain. Among the class I myosins, this protein is distinguished by a long tail domain that is involved in crosslinking actin filaments. This protein localizes to the cytoplasm and may be involved in intracellular movement and membrane trafficking. |
| Mras | This gene encodes a member of the Ras family of small GTPases. These membrane-associated proteins function as signal transducers in multiple processes including cell growth and differentiation, and dysregulation of Ras signaling has been associated with many types of cancer. The encoded protein may play a role in the tumor necrosis factor-alpha and MAP kinase signaling pathways. |
| Bcl2l11 | The protein encoded by this gene contains a Bcl-2 homology domain 3 (BH3). It has been shown to interact with other members of the BCL-2 protein family, including BCL2, BCL2L1/BCL-X(L), and MCL1, and to act as an apoptotic activator. |

The relative gene expression levels are presented as fold change in tables 2 - 9.

**Table 2: Gene expression levels for CsA as determined by qPCR in 5 mice.**

| **Compound** | **CsA** | **CsA** | **CsA** | **CsA** | **CsA** | **MW CsA test** |
|---|---|---|---|---|---|---|
| Gene | *fold change 1 | *fold change 2 | *fold change 3 | *fold change 4 | *fold change 5 | |
| **Bcl2111** | -1,4 | -1,0 | 1,2 | -1,1 | -1,1 | NS |
| **Parp 16** | 1,6 | 1,6 | 1,7 | 1,6 | 2,6 | * |
| **Mras** | 2,0 | 1,7 | 1,8 | 1,6 | 2,3 | * |
| **Myo 1e** | -1,5 | -1,1 | -1,3 | -1,6 | -1,7 | * |
| **Nme6** | 5,4 | 5,5 | 4,0 | 4,4 | 3,7 | * |
| **ABCG5** | -3,5 | -1,8 | 1,0 | -1,3 | 1,2 | NS |
| **ABCG8** | -4,9 | -3,9 | -1,2 | -2,0 | -1,2 | * |
| **SERPINE1** | -4,2 | -3,2 | 1,1 | -1,1 | 2,6 | NS |
| **BAAT** | -5,2 | -4,2 | -1,6 | -2,2 | -1,7 | * |
| **KLF15** | -1,2 | -1,7 | -2,0 | -1,4 | -1,3 | * |

**Table 3: Gene expression levels for CPZ as determined by qPCR in 5 mice.**

| **Compound** | **CPZ** | **CPZ** | **CPZ** | **CPZ** | **CPZ** | **MW CPZ test** |
|---|---|---|---|---|---|---|
| Gene | *fold change 1 | *fold change 2 | *fold change 3 | *fold change 4 | *fold change 5 | |
| **Bcl2111** | -1,2 | -1,4 | -1,2 | -1,9 | -2,3 | * |
| **Parp 16** | -1,1 | -1,4 | -1,1 | -1,4 | -1,0 | * |
| **Mras** | 1,3 | 2,0 | 2,6 | 1,4 | 1,1 | * |
| **Myo 1e** | -1,1 | -1,0 | 1,1 | 1,0 | -1,1 | NS |
| **Nme6** | 1,1 | 1,3 | 1,7 | 1,3 | 1,2 | * |
| **ABCG5** | -4,2 | -2,9 | -6,7 | -3,8 | -1,0 | * |
| **ABCG8** | -2,9 | -2,1 | -5,4 | -4,5 | -1,1 | * |
| **SERPINE1** | 2,9 | -1,4 | -4,7 | -5,8 | -1,8 | NS |
| **BAAT** | -2,3 | -3,3 | -8,5 | -6,4 | 1,4 | * |
| **KLF15** | -2,0 | -1,8 | -3,2 | -1,8 | 1,6 | NS |

**Table 4: Gene expression levels for APAP as determined by qPCR in 5 mice.**

| | **APAP** | **APAP** | **APAP** | **APAP** | **APAP** | **MW APAP test** |
|---|---|---|---|---|---|---|
| Gene | *fold change 1 | *fold change 2 | *fold change 3 | *fold change 4 | *fold change 5 | |
| **Bcl2111** | 1,9 | 1,1 | 2,0 | 2,4 | 1,7 | * |
| **Parp 16** | 1,4 | -1,3 | 1,4 | 1,5 | 1,6 | NS |
| **Mras** | 1,7 | -1,0 | 1,3 | 1,2 | 1,4 | * |
| **Myo 1e** | 1,1 | -1,2 | 1,4 | 1,4 | 1,4 | NS |
| **Nme6** | -1,2 | -1,2 | -1,1 | -1,3 | 1,0 | NS |
| **ABCG5** | -2,1 | -1,3 | 1,4 | -1,4 | 1,1 | NS |
| **ABCG8** | -1,7 | -1,2 | 1,4 | -1,1 | 1,4 | NS |
| **SERPINE1** | -1,6 | -1,4 | 1,2 | -1,3 | 1,5 | NS |
| **BAAT** | -5,0 | -2,0 | -1,4 | -2,8 | -1,5 | * |
| **KLF15** | -1,5 | -1,2 | -1,4 | -1,1 | 1,2 | NS |

**Table 5: Gene expression levels for PQ as determined by qPCR in 5 mice.**

| **Compoun d** | **PQ** | **PQ** | **PQ** | **PQ** | **PQ** | **MW PQ test** |
|---|---|---|---|---|---|---|
| Gene | *fold change 1 | *fold change 2 | *fold change 3 | *fold change 4 | *fold change 5 | |
| **Bcl2111** | 1,3 | 1,1 | 1,1 | 1,2 | 1,1 | NS |
| **Parp 16** | 1,6 | 1,0 | 1,0 | 1,1 | 1,4 | NS |
| **Mras** | 1,4 | 1,3 | 1,1 | 1,1 | 1,4 | NS |
| **Myo 1e** | 1,1 | 1,4 | 1,1 | 1,2 | 1,2 | NS |
| **Nme6** | 1,1 | -1,2 | 1,0 | 1,1 | 1,2 | NS |
| **ABCG5** | 1,1 | 1,1 | 1,7 | -1,3 | -1,6 | NS |
| **ABCG8** | 1,0 | -1,2 | 1,8 | -1,3 | -1,4 | NS |
| **SERPINE 1** | 2,7 | 2,3 | 2,7 | 1,3 | 1,9 | * |
| **BAAT** | -1,2 | -1,0 | 1,7 | 1,1 | -1,4 | NS |
| **KLF15** | 1,3 | -1,0 | 1,3 | 1,1 | -1,2 | NS |

**Table 6: Gene expression levels for ISONZ as determined by qPCR in 5 mice.**

| **Compoun d** | **ISONZ** | **ISONZ** | **ISONZ** | **ISONZ** | **ISONZ** | **MW ISONZ test** |
|---|---|---|---|---|---|---|
| Gene | *fold change 1 | *fold change 2 | *fold change 3 | *fold change 4 | *fold change 5 | |
| **Bcl2111** | 1,9 | 1,7 | 1,3 | -1,2 | -1,2 | NS |
| **Parp 16** | -1,0 | -1,0 | 1,2 | -2,3 | -1,8 | NS |
| **Mras** | 1,7 | -1,1 | 1,3 | -1,3 | -1,3 | NS |
| **Myo 1e** | 1,5 | 1,3 | 1,3 | -1,3 | 1,1 | NS |
| **Nme6** | 1,3 | 1,2 | 1,1 | -1,3 | -1,1 | NS |
| **ABCG5** | 2,0 | 3,0 | 1,1 | 2,0 | 2,0 | * |
| **ABCG8** | 2,1 | 2,8 | 1,0 | 2,3 | 1,9 | NS |
| **SERPINE 1** | 5,4 | 1,9 | 1,4 | -1,4 | -2,9 | NS |
| **BAAT** | 1,8 | 1,7 | 1,1 | -1,3 | 1,3 | NS |
| **KLF15** | 1,2 | 2,4 | 1,0 | 1,1 | 1,2 | NS |

**Table 7: Gene expression levels for TETRA as determined by qPCR in 5 mice.**

| **Compoun d** | **TETRA** | **TETRA** | **TETRA** | **TETRA** | **TETRA** | **MW TETRA test** |
|---|---|---|---|---|---|---|
| Gene | *fold change 1 | *fold change 2 | *fold change 3 | *fold change 4 | *fold change 5 | |
| **Bcl2111** | -1,1 | 1,6 | 1,5 | 1,5 | 1,1 | NS |
| **Parp 16** | 1,0 | 1,1 | 1,1 | 1,0 | -1,1 | NS |
| **Mras** | 1,3 | 1,3 | 1,3 | 1,3 | 1,0 | * |
| **Myo 1e** | 1,0 | 1,2 | -1,1 | 1,1 | 1,1 | NS |
| **Nme6** | 1,2 | -1,4 | -1,4 | 1,1 | 1,0 | NS |
| **ABCG5** | -1,9 | -3,1 | -1,9 | -5,4 | -2,1 | * |
| **ABCG8** | -1,5 | -4,1 | -1,8 | -4,8 | -2,0 | * |
| **SERPINE 1** | -1,6 | 2,4 | 1,1 | -3,2 | -3,0 | NS |
| **BAAT** | -2,2 | -6,1 | -2,7 | -5,5 | -3,1 | * |
| **KLF15** | 1,1 | -1,9 | -1,6 | -2,4 | -1,3 | NS |

**Table 8: Gene expression levels for VA as determined by qPCR in 5 mice.**

| **Compound** | **VA** | **VA** | **VA** | **VA** | **VA** | **MW VA test** |
|---|---|---|---|---|---|---|
| Gene | *fold change 1 | *fold change 2 | *fold change 3 | *fold change 4 | *fold change 5 | |
| **Bcl2111** | -1,0 | -1,2 | -1,2 | -1,4 | -1,3 | * |
| **Parp 16** | -1,1 | -1,5 | -1,8 | -1,4 | -1,4 | * |
| **Mras** | 1,0 | -1,2 | -1,4 | -1,3 | 1,2 | NS |
| **Myo 1e** | -1,1 | -1,3 | -1,5 | -1,1 | -1,3 | * |
| **Nme6** | 1,0 | 1,2 | -1,5 | 1,4 | 1,7 | NS |
| **ABCG5** | 2,0 | -1,7 | -1,1 | 1,4 | 1,6 | NS |
| **ABCG8** | 2,2 | -1,5 | -1,3 | 1,4 | 1,9 | NS |
| **SERPINE1** | 1,5 | -4,0 | -1,3 | -1,3 | 1,5 | NS |
| **BAAT** | 1,3 | 1,0 | -2,0 | 1,4 | 2,5 | NS |
| **KLF15** | 1,0 | -1,9 | -1,7 | -1,1 | 1,1 | NS |

**Table 9: Gene expression levels for AMIOD as determined by qPCR in 5 mice.**

| **Compound** | **AMIOD** | **AMIOD** | **AMIOD** | **AMIOD** | **AMIOD** | **MW AMIOD test** |
|---|---|---|---|---|---|---|
| Gene | *fold change 1 | *fold change 2 | *fold change 3 | *fold change 4 | *fold change 5 | |
| **Bcl2111** | -3,1 | -2,1 | -1,9 | -2,1 | -2,8 | * |
| **Parp 16** | -1,2 | -1,6 | -1,2 | -1,4 | -1,8 | * |
| **Mras** | 1,1 | 1,0 | 1,1 | 1,1 | -1,0 | NS |
| **Myo 1e** | -1,1 | -1,3 | -1,5 | -1,1 | -1,3 | * |
| **Nme6** | 1,3 | -1,4 | -1,1 | -1,1 | 1,1 | NS |
| **ABCG5** | 1,2 | -1,6 | 1,2 | 1,1 | -1,3 | NS |
| **ABCG8** | 2,0 | -1,6 | 1,1 | 1,1 | -1,1 | NS |
| **SERPINE1** | -1,7 | -1,4 | 1,4 | 1,5 | -3,5 | NS |
| **BAAT** | 1,8 | -2,2 | 1,1 | -1,0 | -1,7 | NS |
| **KLF15** | 2,6 | -1,8 | -1,5 | -1,4 | -1,3 | NS |

It is immediately apperant from the data presented above that the cholestatic compounds significantly alter the gene expression of 7 out of 10 marker genes. Under the influence of CsA, genes Parp 16, Mras and Nme are upregulated (i.e. a fold change of > 1) whereas genes Nyo 1 e, ABCG8, BAAT and KLF15 are downregulated (i.e. a fold change of < -1). Remarkably, CPZ induced the upregulation of Mras, Nme and BAAT and the downregulation of Bcl2111, Parp 16, ABCG5 and ABCG8. The control compounds significantly altered the expression of at most 4 of these genes (tables 4 - 9).

It is therefore concluded that the aberrant expression of the 10 marker genes as identified herein are a useful indicator of the cholestatic properties of a compound.

Hence, the invention relates to an in vitro method for determining whether a compound has cholestatic properties, comprising the step of exposing a liver cell to the compound, incubating the liver cell with the compound for an appropriate amount of time, determining the expression level of genes Bcl2l11, Parp 16, Mras, Myo1e, Nme6 ABCG5, ABCG8, SERPINE1, BAAT, and KLF15 in the liver cell, wherein an aberrant expression of more than 4 of these genes indicates that the compound has cholestatic properties.

More than 4 in this respect means at least 5, such as 5, 6 or 7 or more.

Any liver cell will be useful in a method according to the invention, however, precision cut liver slices are preferred. Hence, the invention relates to a method as described above wherein the liver cell is comprised in a mouse precision cut liver slice (PLCS).

The term "aberrant expression" as used herein is meant to indicate that the expression of a gene in a cell exposed to the test compound is increased or decreased in comparison to the expression of the same gene in a cell which is not exposed to the compound. The change in gene expression is usually expressed as fold change or a ratio. A skilled person will appreciate the metes and bounds of these terms. Any method of determining whether a gene is overexpressed or underexpressed will be useful in a method according to the invention, however, qPCR is preferred because of its ease of use and potential to standardize. Moreover, qPCR test for this purpose are commercially available. Therefore, the invention also relates to a method as described above wherein the step of determining the expression level of genes Bcl2l11, Parp 16, Mras, Myo1e, Nme6 ABCG5, ABCG8, SERPINE1, BAAT, and KLF15 is performed by quantitative PCR.

The skilled person will appreciate that any significant fold change may be used in a method according to the invention. In a preferred embodiment, the cut off value for determining whether an expression is increased or decreased is a fixed value, such as a fold change of at least 1,25 or -1,25, such as 1,5 or -1,5. Hence, the invention also relates to a method as decribed above wherein the aberrant expression is an overexpression with a fold change of at least 1.25 or an underexpression with a fold change of at least -1.25.

### Examples

### Example 1: Preparation and culture of liver slices

23-weeks old male C57BL/6 mice were obtained from Harlan (Horst, The Netherlands). Animals were kept for 1 week at a housing temperature of 22°C and at a relative humidity of 30-70%. The lighting cycle was 12-h light and 12-h dark. At the age of 24 weeks animals were sacrificed by an overdose of isoflurane. The treatment protocol was approved by the Ethical Committee for Animal Experiments at Wageningen University.

Immediately after the animals were killed the liver was perfused with PBS and placed in ice-cold Krebs-Henseleit buffer (KHB) (pH 7.4, supplemented with 11 mM glucose). Liver tissue was transported to the laboratory within approximately 30 min and cylindrical liver cores were produced using a surgical biopsy punch with diameter of 5 mm (KAI, SynErgo Europe, Romania). Liver cores were placed in a Krumdieck tissue slicer (Alabama Research and Development, Munford, AL) filled with ice-cold KHB aerated with carbogen and supplemented with 11 mM glucose. Precesion cut liver slices (PCLS) with a diameter of 5 mm, a thickness of 0.2 mm and a weight of approximately 10 mg were prepared. Immediately after preparation, slices were transferred into culture plates filled with pre-warmed (37 degrees C) Williams E medium (WEM). Three liver slices were pre-cultured in one well of the 6-well plate filled with 4 ml of WEM for one hour with continuous shaking (70 rpm). Incubations were performed in an oxygen controlled incubator (Galaxy 48 R New Brunswick, Nijmegen, the Netherlands) at 80% of oxygen; 5% CO2 and the remaining gas volume was filled up to 95% with N2. After one hour of pre-incubation, media were removed and replaced with fresh media containing test compounds or appropriate solvents. After incubations, samples were snap-frozen in liquid nitrogen and stored in -80 °C for further analysis. Samples dedicated to histology were stored in 4 % formaldehyde at room temperature.

### Example 2: Cytotoxicity analysis - dose selection experiments

PCLS were exposed to different model compounds inducing cholestasis, steatosis, necrosis, and to compounds that are not hepatotoxic in vivo. These compounds were selected based on literature search. As pro-cholestatic model compounds CyclosporinA (CsA) and chlorpromazine (CPZ) were selected. For steatosis tetracycline, valproic acid, and amiodarone were selected. As pro-necrotic drugs acetaminophen, paraquat and isoniazid were used.

To select a non-toxic dose used for the exposure experiments, the following concentrations ranges were tested: cyclosporinA 0-80 µM, chlorpromazine 0-80 µM, tetracycline 0-100 µM, valproic acid 0-500 µM, amiodarone 0-100 µM, acetaminophen 0-3000 µM, paraquat 0-10 µM, and isoniazid 0-1000 µM. CyclosporinA, chlorpromazine, tetracycline, amiodarone, acetaminophen, paraquat were dissolved in DMSO, valproic acid was dissolved in ethanol (EtOH), and isoniazid were dissolved in PBS. The compounds were added to the culture medium at a final concentration of 0.1% vol/vol in an appropriate solvent (DMSO, EtOH, or PBS). Slices incubated with the solvents at 0.1 % vol/vol served as controls. Toxicity was assessed with standard biochemical assays indicative for slices viability: LDH, ATP, and protein assay. Dose selection experiments were performed in slices obtained from at least two mice.

### Example 3: PCLS exposure experiments

PCLS were incubated at the same conditions as described above. Slices were exposed for 24 and 48 hours to one concentration of the tested compounds or controls. Concentrations used in the exposure experiments were preselected in the dose selection experiments for each drug individually. The highest concentration that did not cause significant increase of toxicity assessed by biochemical assays (LDH, ATP, and protein assay) was selected. The concentrations used in the exposure experiments were as follows; for the cholestatic exposures 40 µM CsA and 10 uM CPZ. For the steatotic exposures: 40 µM tetracycline, 200 µM valproic acid, and 50 µM amiodarone. For the necrotic compounds: 1000 uM acetaminophen, 5 µM paraquat and 1000 uM isoniazid. Liver slices obtained from five mice were used in five toxic compound or control exposure experiments performed at the same time. Dose selection experiments and exposure experiments were performed at different days. Therefore, ATP, LDH, and protein assays were performed again for the exposure experiments to confirm that the selected doses did also not affect slice viability in these experiments.

### Example 4: ATP and protein measurements

For each ATP and protein measurement a total of three co-cultured slices were placed in 400 µL Cell Lytic MT buffer (Sigma, Zwijndrecht, the Netherlands). In order to compare ATP and protein content in liver slices from different experiments, both ATP and protein were normalized on mass of liver slices. Therefore, the mass was registered for each individual exposure and was for three slices 30 mg ± 25%. Slices were homogenized (6500 g, 8°C) two times for 15 sec using a tissue homogenizer (Precellys 24 Bertin Technologies, Labmakelaar Benelux B.V. Rotterdam, The Netherlands). To remove cellular debris, the homogenates were centrifuged for 5 min (14000 g, 8°C) and the remaining supernatant was divided into two portions of 200 µL. One portion was stored at -80°C for protein measurements and the second 200 µL portion was mixed with 100 µL of ATP lytic buffer (ATPlite, Perkin Elmer, Oosterhout, The Netherlands) for ATP measurements. ATP was measured according to the manufacturer's description using a microplate reader (Synergy TM HT Multi Detection Microplate Reader, Biotek Instruments Inc, Abcoude, the Netherlands) with settings for luminescence: 590/635 nm, top measurement, and sensitivity 230. ATP determination was performed in technical duplicates and luminescence values were recalculated into µM ATP in total liver slices extracts. ATP concentration was normalized on mass of liver slices used for one ATP extraction. ATP levels in toxic compounds exposure experiments are expresses as a relative value compared to control.

The protein concentration was determined according to the Bradford method (Protein assay, BioRad, Veenendaal, The Netherlands). Protein samples of 2 µL were diluted 80 times in PBS and measured according to the manufacturer's instructions. BSA was used as a standard and each measurement was performed in duplicate. The protein concentration was expressed as µg protein per mL of total protein extract. The protein content was normalized on mass of liver slices used for protein extraction and was expressed as a relative protein content compared to protein content measured in control.

### Example 5: Lactate dehydrogenase (LDH) cytotoxicity assay

Lactate dehydrogenase activity in the culture medium of slices was performed according to the manufacturer's protocol (Cytotoxicity Detection Kit (LDH), Roche Diagnostics, Almere, the Netherlands). In brief, culture medium was diluted 5 fold with PBS and mixed with 100 µL reaction mixture from the kit. After 15 minutes of incubation at room temperature, samples were measured at 490 nm using a Synergy TM HT Multi Detection microplate reader. LDH leakage is expressed as a relative value compared to control.

### Example 6: Triglycerides (TG) measurements

Three liver slices cultured together were used to make a homogenate in 300 µL buffer containing 10mM Tris, 1mM EDTA (pH 7.4) and 250 mM sucrose. Aliquots of 10 µL of the liver slices homogenates were used in the assay and measurements were performed according to the manufacturer's protocol (triglycerides liquicolor mono, Instruchemie, Delfzijl, the Netherlands). TG values were measured in mM of total extract and are expressed as a relative value compared to control.

### Example 7: DNA microarray hybridizations

Gene expression analysis in liver slices incubated for 24 hours with the pro-cholestatic, steatotic, and necrotic compounds as well as the controls was performed on the HT Mouse Genome 430 PM array plate(s) using the Affymetrix GeneTitan system. RNA was extracted from three slices cultured together per one exposure experiment using the RNeasy Tissue Mini Kit (Qiagen, Venlo, The Netherlands) according to the manufacturer's instructions. RNA concentration and purity were assessed spectrometrically using a Nano Drop ND-1000 spectrophotometer (Isogen IJsselstein, The Netherlands) by measuring absorption ratios at 260/280 and 230/280 nm. The integrity of the RNA samples was examined using the Shimadzu MultiNA Bioanalyzer (Tokyo, Japan). Biotin- labelled cRNA was generated from high-quality total RNA with the Affymetrix 3'IVT Express Kit (Affymetrix, Santa Clara, CA, USA) according to the manufacturer's specifications with an input of 100 ng total RNA. The Agilent Bioanalyzer (Amstelveen, the Netherlands) and Shimadzu MultiNA Bioanalyzer (Tokyo, Japan) were used to evaluate the quality of cRNA in order to confirm if the average fragment size was according to Affymetrix' specifications. Per sample, 7.5ug cRNA of the obtained biotinylated cRNA samples was fragmented and hybridized in a final concentration of 0.0375 ug /ul on the Affymetrix HT Mouse genome 430 PM array (Affymetrix, Santa Clara, CA, USA. After an automated process of washing and staining by the GeneTitan machine (Affymetrix, Santa Clara, CA, USA) using the Affymetrix HWS kit for Gene Titan, absolute values of expression were calculated from the scanned array using the Affymetrix Command Console v 3.2 software. The data Quality Control was performed in the program Affymetix Expression Console v 1.1 software to determine if all parameters were within quality specifications. The Probe Logarithmic Intensity Error Estimation (PLIER) algorithm method was used for probe summarisation (Qu et al. 2010).

In order to monitor the sample independent control and the performance of each individual sample during hybridization, hybridizations controls were added to the hybridization mixture. The sample dependent controls such as internal control genes, background values, and average signals were used to determine the biological variation between samples. In conclusion, all the data were within data Quality Control thresholds, according to Affymetrix Expression Console specifications. Non-normalized data in a form of the Cell Intensity File (*.CEL) were re-annotated (EntrezGene htmg430pm_Mm_ENTREZG) and the data were RMA normalized (Jetten et al. 2012; Qu et al. 2010).

### Example 8: Biomarkers verification

In order to identify biomarkers indicative for the development of cholestasis, three complementary approaches were applied. In the first approach, DNA microarray data obtained from slices exposed to pro-cholestatic model compounds (CsA and CPZ) were subjected to analysis of variance (ANOVA). Genes with p<0.05 were selected for Venn diagram analysis and genes that were overlapping between the three treatments served as potential biomarkers.

In the second approach, the significant genes identified by GSEA, were used as input for Venn diagram. Similarly to the first approach, genes overlapping between the three treatments were selected as potential biomarkers. Venn analysis was performed using an open access tool http://bioinfogp.cnb.csic.es/tools/venny/index.html.

In the third approach, a random forest was applied. This method was conducted as described previously (Bayjanov et al. 2012). Briefly, genes associating to samples of the two classes (CsA and CPZ) were determined by the random forest based tool PhenoLink using default parameters (Bayjanov et al. 2012). Random forest was used to train 500 decision trees, where each tree is trained on different subsets of the samples and genes. Using the local importance values, a total of 17278 non-informative genes (genes not contributing to distinguish the three classes) were removed. After this step, 28 genes remained that were ranked based on the averaged local importances per class.

Furthermore, biomarkers' expression values (derived from the DNA microarray data) were log2 transformed and median centered followed by hierarchical clustering using default options in Genesis (http://genome.tugraz.at/genesisserver/genesisserver_description.shtml). From this analysis, ten biomarkers were selected.

### Example 9: Biomarkers validation by q-pcr

In further studies, expression of ten candidate biomarkers in PCLS was analyzed by q-pcr using the Biorad CFX96 TM Real-Time Detection System (Bio-Rad, Veenendaal, The Netherlands) with the following cycling conditions: 15 min 95°C followed by 40 cycles of 15s 95 °C and 1 min 60 °C. Reactions were performed in 10 µl and contained 20 ng cDNA, 1x TaqMan PCR Master Mix (Applied Biosystems, Foster City, CA), and depending on the analysed gene, 250 nM probe and 900 nM primer (for actin b) or 1xTaqMan gene expression assay (for the remaining genes) (Applied Biosystems, foster City, CA). Specific primer set for actinβ (ACTB) was developed with Primer Express 1.5 (Applied Biosystems, Nieuwerkerk aan den IJssel, The Netherlands) and sequences were as follows, probe: TGT CCC TGT ATG CCT CTG GTC GTA CCA C, forward primer: AGC CAT GTA CGT AGC CAT CCA, and reverse primer: TCT CCG GAG TCC ATC ACA ATG (Szalowska et al. 2011). Primers for Kruppel-like factor 15 (KLF15), bile acid-CoenzymeA:amino acid N-acyltransferase (BAAT), serine(or cysteine) peptidase inhibitor clade E, member 1 (SERPINE1/PAI1), ATP-binding cassette, sub-family G (WHITE), member 8 (ABCG8), ATP-binding cassette, sub-family G (WHITE), member 5 (ABCG5), non-metastatic cells 6, protein expressed in (nucleoside-diphosphate kinase) (Nme), poly (ADP-ribose)polymerase family, member 16 (Parp16), muscle and microspikes RAS (Mras), myosinIE (Myo1e), and BCL2-like 11 (Bcl2l11) were purchased from Applied Biosystems.

The numbers of assays from Applied Biosystems are given in Table 10.

**Table 10: Applied Biosystems assay numbers.**

| **Applied Biosystems assay nr** | **Gene name** | **Gene symbol** |
|---|---|---|
| Mm00517792_m1 | Kruppel-like factor 15 | KLF15 |
| Mm00476075_m1 | bile acid-CoenzymeA:amino acid N-acyltransferase | BAAT |
| Mm00435860_m1 | serine(or cysteine) peptidase inhibitor clade E, member 1 | SERPINE1 |
| Mm00445970_m1 | ATP-binding cassette, sub-family G (WHITE), member 8 | ABCG8 |
| Mm00446241_m1 | ATP-binding cassette, sub-family G (WHITE), member 5 | ABCG5 |
| Mm00444447_m1 | non-metastatic cells 6, protein expressed in (nucleoside-diphosphate kinase) | Nme6 |
| Mm00838806_m1 | poly (ADP-ribose)polymerase family, | Parp16 |
| | member 16 | |
| Mm00619299_m1 | myosinIE | Myo1e |
| Mm00485144_m1 | muscle and microspikes RAS | Mras |
| Mm00437796_m1 | BCL2-like 11 (apoptisis facilitator) | Bcl2l11 |

Data were analyzed with SDS 2.0 software (Bio-Rad). For each sample, the RT-PCR reaction was performed in triplicate and the averages of the obtained threshold cycle values (CT) were processed for further calculations. To check for contaminating DNA, samples without reverse transcriptase (-RT reaction) were analysed as well. For normalization ACTB was used. Relative expression was calculated with Δ (Δ (CT))-method (Livak and Schmittgen 2001).

### Example 10: Statistical analysis

Mann-Whitney test was used to calculate differences between controls and slices incubated with the tested compounds for ATP, protein, LDH, TG, and bile acids. The cut off for statistical significance was set at a p-value < 0.05.

Mann-Whitney - test was used to determine if there was a significant difference in gene expression assessed by q-pcr in slices exposed to different compounds. A p-value <0.05 was considered significant.

### References

1. Abernathy, C. O., Zimmerman, H. J., Ishak, K. G., Utili, R., and Gillespie, J. (1992). Drug-induced cholestasis in the perfused rat liver and its reversal by tauroursodeoxycholate: an ultrastructural study. Proc. Soc. Exp. Biol. Med. 199(1), 54-58.
2. Akerboom, T., Schneider, I., vom, D. S., and Sies, H. (1991). Cholestasis and changes of portal pressure caused by chlorpromazine in the perfused rat liver. Hepatology 13(2), 216-221.
3. Ansede, J. H., Wright, M. R., St, C. R., III, Hart, R. W., Gefroh, H. A., and Brouwer, K. R. (2009). Characterization of sandwich-cultured hepatocytes as an in vitro model to assess the hepatobiliary disposition of copper. Drug Metab Dispos. 37(5), 969-976.
4. Antherieu, S., Azzi, P. B., Dumont, J., bdel-Razzak, Z., Guguen-Guillouzo, C., Fromenty, B., Robin, M. A., and Guillouzo, A. (2012). Oxidative stress plays a major role in chlorpromazine-induced cholestasis in human heparg cells. Hepatology*.*
5. Auli, M., Domenech, A., Andres, A., Orta, M., Salva, M., Descotes, J., and Prats, N. (2012). Multiparametric immunotoxicity screening in mice during early drug development. Toxicol. Lett. 214(2), 200-208.
6. Barth, A., Braun, J., and Muller, D. (2006). Influence of Verapamil and Cyclosporin A on bile acid metabolism and transport in rat liver slices. Exp. Toxicol. Pathol. 58(1), 31-37.
7. Bayjanov, J. R., Molenaar, D., Tzeneva, V., Siezen, R. J., and van Hijum, S. A. (2012). PhenoLink--a web-tool for linking phenotype to -omics data for bacteria: application to gene-trait matching for Lactobacillus plantarum strains. BMC. Genomics 13, 170.
8. Blomme, E. A., Yang, Y., and Waring, J. F. (2009). Use of toxicogenomics to understand mechanisms of drug-induced hepatotoxicity during drug discovery and development. Toxicol. Lett. 186(1), 22-31.
9. Carlton, V. E., Pawlikowska, L., and Bull, L. N. (2004). Molecular basis of intrahepatic cholestasis. Ann. Med. 36(8), 606-617.
10. Chan, F. K., Zhang, Y., Lee, S. S., and Shaffer, E. A. (1998). The effects of liver transplantation and cyclosporine on bile formation and lipid composition: an experimental study in the rat. J. Hepatol. 28(2), 329-336.
11. Chanussot, F., and Benkoel, L. (2003). Prevention by dietary (n-6) polyunsaturated phosphatidylcholines of intrahepatic cholestasis induced by cyclosporine A in animals. Life Sci. 73(4), 381-392.
12. Dandel, M., Lehmkuhl, H. B., Knosalla, C., and Hetzer, R. (2010). Impact of different long-term maintenance immunosuppressive therapy strategies on patients' outcome after heart transplantation. Transpl. Immunol. 23(3), 93-103.
13. de Graaf, I., Olinga, P., de Jager, M. H., Merema, M. T., de, K. R., van de Kerkhof, E. G., and Groothuis, G. M. (2010). Preparation and incubation of precision-cut liver and intestinal slices for application in drug metabolism and toxicity studies. Nat. Protoc. 5(9), 1540-1551.
14. Delgado, T. C., Barosa, C., Nunes, P. M., Scott, D. K., O'Doherty, R. M., Cerdan, S., Geraldes, C. F., and Jones, J. G. (2012). Effect of cyclosporine A on hepatic carbohydrate metabolism and hepatic gene expression in rat. Expert. Opin. Drug Metab Toxicol. 8(10), 1223-1230.
15. Deters, M., Strubelt, O., and Younes, M. (1997). Reevaluation of cyclosporine induced hepatotoxicity in the isolated perfused rat liver. Toxicology 123(3), 197-206.
16. Elferink, M. G., Olinga, P., Draaisma, A. L., Merema, M. T., Bauerschmidt, S., Polman, J., Schoonen, W. G., and Groothuis, G. M. (2008). Microarray analysis in rat liver slices correctly predicts in vivo hepatotoxicity. Toxicol. Appl. Pharmacol. 229(3), 300-309.
17. Fujimura, H., Murakami, N., Kurabe, M., and Toriumi, W. (2009). In vitro assay for drug-induced hepatosteatosis using rat primary hepatocytes, a fluorescent lipid analog and gene expression analysis. J. Appl. Toxicol. 29(4), 356-363.
18. Geier, A., Fickert, P., and Trauner, M. (2006). Mechanisms of disease: mechanisms and clinical implications of cholestasis in sepsis. Nat. Clin. Pract. Gastroenterol. Hepatol. 3(10), 574-585.
19. Groot, M. J., Ossenkoppele, J. S., Bakker, R., Pfaffl, M. W., Meyer, H. H., and Nielen, M. W. (2007). Reference histology of veal calf genital and endocrine tissues - an update for screening on hormonal growth promoters. J. Vet. Med. A Physiol Pathol. Clin. Med. 54(5), 238-246.
20. Hadzic, N., Bull, L. N., Clayton, P. T., and Knisely, A. S. (2012). Diagnosis in bile acid-CoA: amino acid N-acyltransferase deficiency. World J. Gastroenterol. 18(25), 3322-3326.
21. Halilbasic, E., Claudel, T., and Trauner, M. (2012). Bile acid transporters and regulatory nuclear receptors in the liver and beyond. J. Hepatol*.*
22. Hoshi, K., and Fujino, S. (1992). Difference between effects of chlorpromazine and perphenazine on microsomal phospholipids and enzyme activities in rat liver. J. Toxicol. Sci. 17(2), 69-79.
23. Ikegami, T., and Matsuzaki, Y. (2008). Ursodeoxycholic acid: Mechanism of action and novel clinical applications. Hepatol. Res. 38(2), 123-131.
24. Jelier, R., Schuemie, M. J., Veldhoven, A., Dorssers, L. C., Jenster, G., and Kors, J. A. (2008). Anni 2.0: a multipurpose text-mining tool for the life sciences. Genome Biol. 9(6), R96.
25. Jetten, M. J., Gaj, S., Ruiz-Aracama, A., de Kok, T. M., van Delft, J. H., Lommen, A., van Someren, E. P., Jennen, D. G., Claessen, S. M., Peijnenburg, A. A., Stierum, R. H., and Kleinjans, J. C. (2012). 'Omics analysis of low dose acetaminophen intake demonstrates novel response pathways in humans. Toxicol. Appl. Pharmacol. 259(3), 320-328.
26. Kamisako, T., and Ogawa, H. (2005). Alteration of the expression of adenosine triphosphate-binding cassette transporters associated with bile acid and cholesterol transport in the rat liver and intestine during cholestasis. J. Gastroenterol. Hepatol. 20(9), 1429-1434.
27. Kass, G. E. (2006). Mitochondrial involvement in drug-induced hepatic injury. Chem. Biol. Interact. 163(1-2), 145-159.
28. Khan, A. A., Chow, E. C., Porte, R. J., Pang, K. S., and Groothuis, G. M. (2011). The role of lithocholic acid in the regulation of bile acid detoxication, synthesis, and transport proteins in rat and human intestine and liver slices. Toxicol. In Vitro 25(1), 80-90.
29. Lefebvre, P., Cariou, B., Lien, F., Kuipers, F., and Staels, B. (2009). Role of bile acids and bile acid receptors in metabolic regulation. Physiol Rev. 89(1), 147-191.
30. Lindberg, M. C. (1992). Hepatobiliary complications of oral contraceptives. J. Gen. Intern. Med. 7(2), 199-209.
31. Livak, K. J., and Schmittgen, T. D. (2001). Analysis of relative gene expression data using real-time quantitative PCR and the 2(-Delta Delta C(T)) Method. Methods 25(4), 402-408.
32. Mackay, A. V., Healey, A. F., and Baker, J. (1974). The relationship of plasma chlorpromazine to its 7-hydroxy and sulphoxide metabolites in a large population of chronic schizophrenics. Br. J. Clin. Pharmacol. 1 (5), 425-430.
33. Malhi, H., and Kaufman, R. J. (2011). Endoplasmic reticulum stress in liver disease. J. Hepatol. 54(4), 795-809.
34. Muench, J., and Hamer, A. M. (2010). Adverse effects of antipsychotic medications. Am. Fam. Physician 81(5), 617-622.
35. Oo, Y. H., Shetty, S., and Adams, D. H. (2010). The role of chemokines in the recruitment of lymphocytes to the liver. Dig. Dis. 28(1), 31-44.
36. Padda, M. S., Sanchez, M., Akhtar, A. J., and Boyer, J. L. (2011). Drug-induced cholestasis. Hepatology 53(4), 1377-1387.
37. Paulen, M. E., Folger, S. G., Curtis, K. M., and Jamieson, D. J. (2010). Contraceptive use among solid organ transplant patients: a systematic review. Contraception 82(1), 102-112.
38. Qu, Y., He, F., and Chen, Y. (2010). Different effects of the probe summarization algorithms PLIER and RMA on high-level analysis of Affymetrix exon arrays. BMC. Bioinformatics. 11, 211.
39. Rezzani, R. (2006). Exploring cyclosporine A-side effects and the protective role-played by antioxidants: the morphological and immunohistochemical studies. Histol. Histopathol. 21 (3), 301-316.
40. Rotolo, F. S., Branum, G. D., Bowers, B. A., and Meyers, W. C. (1986). Effect of cyclosporine on bile secretion in rats. Am. J. Surg. 151(1), 35-40.
41. Setchell, K. D., Schwarz, M., O'Connell, N. C., Lund, E. G., Davis, D. L., Lathe, R., Thompson, H. R., Weslie, T. R., Sokol, R. J., and Russell, D. W. (1998). Identification of a new inborn error in bile acid synthesis: mutation of the oxysterol 7alpha-hydroxylase gene causes severe neonatal liver disease. J. Clin. Invest 102(9), 1690-1703.
42. Shin, M., Asada, S., Mizumori, N., Sano, K., and Umezawa, C. (1998). Effect of thioridazine or chlorpromazine on increased hepatic NAD+ level in rats fed clofibrate, a hypolipidaemic drug. J. Pharm. Pharmacol. 50(4), 431-436.
43. Sinal, C. J., Tohkin, M., Miyata, M., Ward, J. M., Lambert, G., and Gonzalez, F. J. (2000). Targeted disruption of the nuclear receptor FXR/BAR impairs bile acid and lipid homeostasis. Cell 102(6), 731-744.
44. Subramanian, A., Tamayo, P., Mootha, V. K., Mukherjee, S., Ebert, B. L., Gillette, M. A., Paulovich, A., Pomeroy, S. L., Golub, T. R., Lander, E. S., and Mesirov, J. P. (2005). Gene set enrichment analysis: a knowledge-based approach for interpreting genome-wide expression profiles. Proc. Natl. Acad. Sci. U. S. A 102(43), 15545-15550.
45. Szalowska, E., Meijer, K., Kloosterhuis, N., Razaee, F., Priebe, M., and Vonk, R. J. (2011). Sub-chronic administration of stable GIP analog in mice decreases serum LPL activity and body weight. Peptides 32(5), 938-945.
46. Thoolen, B., Maronpot, R. R., Harada, T., Nyska, A., Rousseaux, C., Nolte, T., Malarkey, D. E., Kaufmann, W., Kuttler, K., Deschl, U., Nakae, D., Gregson, R., Vinlove, M. P., Brix, A. E., Singh, B., Belpoggi, F., and Ward, J. M. (2010). Proliferative and nonproliferative lesions of the rat and mouse hepatobiliary system. Toxicol. Pathol. 38(7 Suppl), 5S-81S.
47. Van Dyke, R. W., and Scharschmidt, B. F. (1987). Effects of chlorpromazine on Na+-K+-ATPase pumping and solute transport in rat hepatocytes. Am. J. Physiol 253(5 Pt 1), G613-G621.
48. Van Summeren, S. A., Renes, J., Bouwman, F. G., Noben, J. P., van Delft, J. H., Kleinjans, J. C., and Mariman, E. C. (2011). Proteomics investigations of drug-induced hepatotoxicity in HepG2 cells. Toxicol. Sci. 120(1), 109-122.
49. Van Summeren, S. A., Renes, J., van Delft, J. H., Kleinjans, J. C., and Mariman, E. C. (2012). Proteomics in the search for mechanisms and biomarkers of drug-induced hepatotoxicity. Toxicol. In Vitro 26(3), 373-385.
50. Velayudham, L. S., and Farrell, G. C. (2003). Drug-induced cholestasis. Expert. Opin. Drug Saf 2(3), 287-304.
51. Werle-Schneider, G., Wolfelschneider, A., von Brevern, M. C., Scheel, J., Storck, T., Muller, D., Glockner, R., Bartsch, H., and Bartelmann, M. (2006). Gene expression profiles in rat liver slices exposed to hepatocarcinogenic enzyme inducers, peroxisome proliferators, and 17alpha-ethinylestradiol. Int. J. Toxicol. 25(5), 379-395.
52. Wolf, A., Trendelenburg, C. F., ez-Fernandez, C., Prieto, P., Houy, S., Trommer, W. E., and Cordier, A. (1997). Cyclosporine A-induced oxidative stress in rat hepatocytes. J. Pharmacol. Exp. Ther. 280(3), 1328-1334.
53. Woolbright, B. L., and Jaeschke, H. (2012). Novel insight into mechanisms of cholestatic liver injury. World J. Gastroenterol. 18(36), 4985-4993.
54. Zhao, Y., Zhai, D., He, H., Liu, J., Li, T., Chen, X., and Ji, H. (2009). Matrine improves 17alpha-ethinyl estradiol-induced acute cholestasis in rats. Hepatol. Res. 39(11), 1144-1149.
55. Zhou, Z. X., Wang, T., Jiang, Z. Z., Shang, J., Liu, L., Zhang, Y., Zhu, D., Huang, X., Zhang, S., Hu, Y., Wang, J. Y., and Zhang, L. Y. (2011). Beneficial effects of colchicine on 17alpha-ethynylestradiol-induced cholestasis in rats. Arzneimittelforschung. 61 (3), 173-179.
56. Zollner, G., Marschall, H. U., Wagner, M., and Trauner, M. (2006). Role of nuclear receptors in the adaptive response to bile acids and cholestasis: pathogenetic and therapeutic considerations. Mol. Pharm. 3(3), 231-251.
57. Zollner, G., Wagner, M., and Trauner, M. (2010). Nuclear receptors as drug targets in cholestasis and drug-induced hepatotoxicity. Pharmacol. Ther. 126(3), 228-243.

## Claims

1. In vitro method for determining whether a compound has cholestatic properties, comprising the step of exposing a liver cell to the compound, incubating the liver cell with the compound for an appropriate amount of time, determining the expression level of genes Bcl2l11, Parp 16, Mras, Myo1e, Nme6 ABCGS, ABCG8, SERPINE1, BAAT, and KLF15 in the liver cell, wherein an aberrant expression of more than 4 of these genes indicates that the compound has cholestatic properties.

2. In vitro method according to claim 1 wherein the liver cell is comprised in a mouse precision cut liver slice (PLCS).

3. Method according to claims 1 or 2 wherein the step of determining the expression level of genes Bcl2l11, Parp 16, Mras, Myo1e, Nme6 ABCG5, ABCG8, SERPINE1, BAAT, and KLF15 is performed by quantitative PCR.

4. Method according to any one of claims 1 - 3 wherein the aberrant expression is an overexpression with a ratio of at least 1.25 or an underexpression with a ratio of at least 0,75.
